# EUROPEAN PATENT SPECIFICATION

(11) **EP 3 971 563 B1**
(45) Date of publication and mention of the grant of the patent: **14.02.2024**
(21) Application number: 20197295.7
(22) Date of filing: 21.09.2020
(51) Int. Cl.: G01N 27/407

(54) **METHOD FOR MANUFACTURING AN ELECTROCHEMICAL GAS SENSOR**
VERFAHREN ZUR HERSTELLUNG EINES ELEKTROCHEMISCHEN GASSENSORS
PROCÉDÉ DE FABRICATION D'UN CAPTEUR DE GAZ ÉLECTROCHIMIQUE

(43) Date of publication of application: 23.03.2022
(73) Proprietor: Sensirion AG, 8712 Stäfa (CH)
(72) Inventor: Röck, Frank, 8712 Stäfa (CH); Gubser, Marc, 8712 Stäfa (CH); Daix, Nicolas, 8712 Stäfa (CH); Bürgi, Lukas, 8712 Stäfa (CH); Hummel, René, 8712 Stäfa (CH)
(74) Representative: Toleti, Martin

(56) References cited:
- EP-A1- 2 896 962
- US-A1- 2003 205 078
- US-A1- 2008 053 827
- US-A1- 2013 075 255
- US-B1- 6 270 651
- VELASCO G ET AL: "THIN SOLID STATE ELECTROCHEMICAL GAS SENSORS", SENSORS AND ACTUATORS, ELSEVIER SEQUOIA S.A. LAUSANNE, CH, vol. 2, 1 January 1982 (1982-01-01), pages 371-384, XP001106616, DOI: 10.1016/0250-6874(81)80057-1

## Description

### Technical Field

The present invention relates to a method for manufacturing an electrochemical gas sensor.

### Background Art

An electrochemical gas sensor includes at least two electrodes and an electrolyte between the electrodes. A first of the electrodes is referred to as sensing electrode or working electrode. It is in contact not only with the electrolyte but also with a medium to be investigated, e.g. ambient air. The medium may hold a target gas which target gas is desired to be detected by the electrochemical gas sensor as to its presence and / or concentration in the medium. A second of the electrodes is referred to as reference electrode. The reference electrode is in contact with the electrolyte but is out of contact with the medium to be investigated. When approaching the sensing electrode, an oxidation or reduction reaction involving the target gas in the medium takes place at the sensing electrode. Ions resolved from the sensing electrode in response to the electrochemical reaction with the target gas transit the ion-conducting electrolyte towards the reference electrode and chemically interact there. These ions are called mobile ions. An electrical current and / or voltage is generated between the sensing electrode and the reference electrode, in response to the electrochemical reactions.

A majority of electrochemical gas sensors are based on a solid, ion-conducting electrolyte that separates the target gas from the reference electrode. All commercially available electrochemical CO2 sensors are based on solid electrolytes. They are based on a sintered, solid state ion conductor as electrolyte with a thickness of several 100 µm.

Different solid state electrolytes are available, but in general the choice is limited. All of the currently used and investigated solid state electrolytes suffer from at least one, but frequently several of the following drawbacks:
- A high operation temperature is required to obtain sufficient ionic conductivity, such as 500° C or more.
- High processing temperatures are required during production, such as 1100° C or more.
- Instability in view of humidity.
- Stoichiometry is complex.
- Incompatibility of the thermal expansion coefficient with the rest of the sensor system.
- Incompatibility with established chemical and physical deposition processes.

As to the thermal incompatibility of the various components in the sensor system, it is noted that all of the commercially available products use an alumina substrate which typically is dominant in terms of thermal expansion coefficients. As to the incompatibility with established chemical and physical deposition processes, it is noted that due to the complexity of the stoichiometry of the known solid electrolytes it is very difficult to obtain the desired stoichiometry after depositing a thin film by CVD, ALD or PVD. In many applications, the mobile ion is lithium or sodium which have a higher diffusivity than other ions/atoms/molecules used in the solid electrolytes. Therefore, these ions are underrepresented in the thin film after deposition and/or after processing these thin films at temperatures of more than 600°C because of a disadvantageous surface-to-volume ratio. Any of these drawbacks limits a degree of freedom in design significantly and has a direct impact on the sensor performance.

Accordingly, it is desired to provide an electrochemical gas sensor with a solid electrolyte avoiding one or more of the above drawbacks.

EP 2 896 962 A1 discloses a gas sensor chip comprising a substrate, a cavity in the substrate, and a membrane extending over the cavity. Furthermore, the gas sensor chip comprises an arrangement comprising a thin-film solid material, wherein the thin-film solid material is arranged between a reference electrode and a sensing electrode, and a heating element arranged in or on the membrane, for heating the thin-film solid material, the reference electrode, and the sensing electrode. The arrangement is arranged on a front side of the membrane or on a reverse side of the membrane within the cavity, wherein the thin-film solid material has a Li-ions conducting garnet-like crystal structure.

VELASCO G ET AL: «THIN SOLID STATE ELECTROCHEMICAL GAS SENSORS", SENSORS AND ACTUATORS, vol. 2, 1 January 1982, pp. 371-384, DOI: 10.1016/0250-6874(81)80057-1 discloses a new family of electrochemical gas sensors based on the use of thin and thick film technologies. This microelectronics approach allowed them to design solid state devices for oxygen and hydrogen detection. Their experimental results evince two major interests, besides those associated with sensor miniaturization, lower price, reliability, etc.: First, wide variety of available materials and substances, local modification of their composition and associated properties using techniques such as thermal diffusion and ion implantation.
Second, possible creation of complex functions using different multi-layered geometries; for example, the catalytic activity of a platinum electrode, for the CO/O2 reaction, may be modified by changing thickness, texture and partial capping of this electrode. Two types of new sensors using this approach are described: a zirconia-based oxygen sensor for combustion control and a hydrogen sensor working at room temperature.

US 2008/053827 A1 discloses an equilibrium potential gas sensor comprising a structure including a reference pole having a reference material and an electronic conductive material and a sensing pole having a sensing material and an electronic conductive material provided through a solid electrolyte in which the said solid electrolyte, the said reference material and the said sensing material are constituted of lithium ionic conductors and a method of manufacturing the same. The gas sensor employs a solid electrolyte operable at a low temperature not more than 200° C., particularly at room temperature, not influenced by moisture or the like and excellent in age-based stability with high measurement sensitivity as well as high responsibility.

### Disclosure of the Invention

This problem is solved by a method for manufacturing an electrochemical gas sensor according to claim 1. The electrochemical gas sensor is configured to sense a target gas in a medium applied to the gas sensor. The medium may include a carrier gas, such as ambient air. The target gas preferably is one or more of CO₂, O₂, NOx, and SOx. Accordingly, the electrochemical gas sensor may be used for measuring a presence and / or a concentration of one or more of the listed target gases in a carrier gas.

In conventional solid state electrochemical gas sensors the electrolyte used is deposited as an ion-conducting thick film. I.e. the ion-conducting electrolyte material is deposited onto the reference electrode during manufacturing as a thick film of several 100 µm, and, of course, remains ion-conducting after manufacture of the gas sensor. Instead, now, a non-ion-conducting electrolyte material is deposited as a thin film onto the reference electrode, with a thickness s of the thin film with s ≤ 5um, and even more preferably with s ≤ 1pm.

Hence, the conventional solid state, thick ion-conducting electrolyte material requiring cumbersome manufacturing and deposition techniques is replaced by a thin layer of a non-conducting - i.e. electronically and also ionically non-conducting - ceramic or glass, preferably deposited by one of CVD (Chemical Vapor Deposition), ALD (Atomic Layer Deposition) or PVD (Physical Vapor Deposition). By this means, one of the standard deposition processes can be used. Only after deposition of the ionically non-conducting material i.a. this material is turned into an ionically conducting electrolyte.

The following criteria result in the specified thickness of the thin film: The thin film preferably is thick enough to separate the reference electrode from the ambient atmosphere at operation temperature, which preferably is a temperature between 250°C and 500°C. And the thin film preferably is thin enough to allow the mobile species, i.e. ions released from a sensing electrode, to traverse the thin film material during operation at elevated temperatures such as at the operation temperature.

The actual thickness strongly depends on the material selected for the thin film, and, even more important, depends on one or more of its conformal deposition ability, its density, its impurity content/defect density and therefore its exact stoichiometry.

Furthermore, it is also preferred to combine more than one layer of an initially non-ion-conducting material in order to tune the properties of the whole gas sensor system, which properties may include one or more of stress, adhesion, etc.

Accordingly, it is envisaged that in the method for manufacturing the gas sensor, a semi-manufactured gas sensor is provided including a non-ion-conducting thin film of the above thickness s. This semi-manufactured gas sensor comprises at least a substrate, a reference electrode, the non-ion-conducting thin film of glass or ceramic, and a sensing electrode, also referred to as working electrode. Hence, an arrangement comprising the thin film, the sensing electrode and the reference electrode is supported by the substrate with the reference electrode facing the substrate. Preferably, the reference electrode is completely encapsulated by the substrate and the thin film without any exposure to the ambient, and preferably, the thin film material is completely confined by the sensing electrode, the reference electrode, and the substrate. Instead, the sensing electrode is exposed to the medium to be investigated, e.g. to ambient air, in order to chemically interact with the target gas if present in the medium. The sensing electrode is porous. The porosity is such that the target gas can reach the thin film electrolyte. The aforementioned chemical reaction takes place at the interface of electrolyte and sensing electrode. Sometimes this interface is referred to as "triple phase boundary", consisting of electrolyte, target gas, and sensing electrode. Hence, the sensing electrode may also be considered as permeable for at least the target gas.

The sensing electrode comprises a salt the cation of which is the mobile ion in the operation of the gas sensor, and the reference electrode preferably comprises a noble metal that can form chemical compounds with the mobile ions.

In a subsequent manufacturing step, i.e. at least after deposition of the arrangement including the reference electrode, the non-ion-conducting thin film and the sensing electrode, at least this arrangement is heated to or above an annealing temperature at which annealing temperature the thin film becomes irreversibly ion-conducting. The mechanism responsible for turning the thin film into an ion-conductor, in response to heating to the annealing temperature, may comprise diffusion and intercalation of mobile ions resolved from the sensing electrode into the thin film, and/or transformation of the thin film into a ion-conducting compound, and/or formation of a percolating path of ion-conducting compounds in the thin film, and/or formation of an interphase between the thin film and the reference electrode, and/or formation of an interphase between the thin film and the sensing electrode. After annealing - including a subsequent cooling down to ambient temperature -, the thin film irreversibly remains ion-conducting, i.e. it remains long-term ion-conducting during the envisaged lifetime of the gas sensor operation. Hence, a heating step is applied to a combination of the reference electrode, the originally non-ion-conducting thin film material which is a ceramic or a glass material, and which shall serve as electrolyte after annealing, and the sensing electrode. In response to the treatment with sufficient heat when arranged between the sensing electrode and the reference electrode, mobile ions released from the sensing electrode transit into the thin film material and make this material becomes ion-conducting. This process is also referred to as incorporation of the mobile ions into the thin film material. Afterwards, the thin film can serve as electrolyte in a gas sensor application.

Preferably, the annealing temperature is greater than or equal to 300°C, preferably greater than or equal to 400°C, preferably between 400°C and 500°C. In one embodiment, an oven or any other heating means external to the gas sensor may generate the annealing temperature. In a different embodiment, a heater integrated in the gas sensor, and preferably arranged in or on the substrate of the gas sensor, may arrange for the annealing temperature. This heater preferably is a resistive heater in or on the substrate, and preferably is electrically controllable. The substrate preferably is a semiconductor substrate, such as a silicon substrate.

In both embodiments, at least the thin film, the reference electrode, and the sensing electrode are heated to at least the annealing temperature. However, more likely the entire gas sensor is heated to at least the annealing temperature. The annealing temperature may depend from the materials used for the thin film, the sensing electrode and the reference electrode. Summarizing, in the semi-manufactured gas sensor the thin film is an electronically non-conducting and ionically non-conducting ceramic or glass. However, after heating the semi-manufactured gas sensor to or above the annealing temperature, the thin film is turned into an ion-conducting state from the previous non-ion-conducting state by means of mobile ions being released from the sensing electrode in response to the heating / annealing of the arrangement.

According to an example that is not part of the present invention, an electrochemical gas sensor is provided as a result of the above manufacturing process. According to a further example that is not part of the present invention, an electrochemical gas sensor is provided independent from the above manufacturing method, which comprises a substrate and an arrangement supported by the substrate, which arrangement comprises a sensing electrode porous for the target gas and configured to chemically interact with the target gas, a reference electrode facing the substrate, and a thin film arranged between the sensing electrode and the reference electrode. The thin film - given its post-annealing status - is an ionically conducting ceramic or glass, for conducting ions released from and accepted by the sensing electrode in response to an electrochemical reaction with the target gas. A thickness s of the thin film is s ≤ 5um, and even more preferably is s ≤ 1um. In some embodiments, even a thickness s of the thin film between 80 nm and 500 nm can be achieved, and in particular between 100 nm and 400 nm, in particular by applying one of the depositing methods of CVD, ALD, or PVD.

Advantages of the method include that the manufacturing process of a solid-state electrochemical gas sensor now is significantly simplified by giving more flexibility in the choice of materials used and by applying one of the established thin film deposition methods, including but not limited to CVD, ALD or PVD. This enables a high volume production with established manufacturing methods and compatibility with MEMS technology. The reduced thickness of the thin film glass or ceramic electrolyte compared to state of the art gas sensors allows lower operating temperatures during operation of the gas sensor, because, first, a diffusion length of ions resolved from the sensing electrode - such as of lithium ions or sodium ions or other ions - is much shorter than in thick film solid electrolytes in the state of the art. A diffusion length of less than 1 um may be achieved by the thin film compared to several 100 um in the thick film electrolytes in the state of the art. Second, the operating power can be reduced in view of the lower volume of the thin film to be heated in comparison with the conventional thick film structures. These structures e.g. were deposited by screen printing, drop coating or ink jetting. Additionally, those technologies have their limits in miniaturization, and structures with a lateral dimension < 200 um are difficult to realize. In addition, annealing temperatures exceeding 500°C by far had to be used.

According to the invention, a material for the thin film is selected from the group consisting of SiₓO_{y}, SiₓN_{y}, SiOₓN_{y}, Al₂O₃, BaZrO₃, and LaAlO₃, or any combination thereof.Hence, besides SiₓO_{y} other intrinsically non-conductive materials, or a combination thereof could be used, such as Si3N4. Any of these materials were found to become ion-conducting in response to the application of heat when arranged between the sensing electrode and the reference electrode, i.e. in response to being exposed to a material specific annealing temperature, while initially these materials are non-ion-conducting.

The reference electrode preferably consists of or comprises a noble metal, in particular platinum or gold. The sensing electrode chemically interacts with the target gas. For this reason, a material or compound is selected for the sensing electrode which includes a salt the anion of which is adapted to the target gas. This adaptation is to be understood in the following way. The salt of the sensing electrode preferably can be characterized or determined as a product of an electrochemical reaction involving only the target gas and the mobile ion, and optionally one or more of oxygen and water. This enables the gas sensor to convert an electrochemical reaction of the target gas with the material of the sensing electrode into an electrical measure such as the electric current between the sensing electrode and the reference electrode in response to the ion flow from the sensing electrode to the reference electrode through the thin film. Hence, the compound of the sensing electrode is adapted to release the cations, wherein the compound is selected from the group consisting of sodium salts, lithium salts, sodium hydroxides, and lithium hydroxides. Preferably, the sensing electrode also comprises noble metal, in particular as a powder, in particular gold nanoparticles. The cations of these various salts were found to penetrate into the thin film made from ceramic or glass. For the specific target gas of CO₂ the salt preferably is selected to comprise a carbonate anion. For the specific target gas of SOx the salt preferably is selected to comprise a sulfate anion. For the specific target gas of NOx the salt preferably is selected to comprise a nitrate/nitrite anion. Preferably, in the sensing electrode the ratio of noble metal to salt is such that after the annealing step the noble metal provides a percolation path for electrons
from the thin film to a conductor contacting the sensing electrode. In a preferred embodiment of the sensing electrode, the noble metal comprises a gold powder and the salt comprises a lithium salt, and the ratio of gold powder to lithium salt is at least 10:1 by weight.

By decreasing the thickness of the electrolyte by three orders of magnitude compared to the state of the art, the roughness of its surface will decrease as well and consequently a direct adhesion of the sensing electrode to the electrolyte thin film may suffer. This is especially the case if primary particles used for manufacturing the sensing electrode are in the dimensional range of or even larger than the thickness / roughness of the thin film, and/or if the annealing temperature used is below a melting point of the used sensing electrode materials.

Further, the thinner the elements of a solid-state electrochemical gas sensors are, which elements may include a heater, the reference electrode, the solid electrolyte and the sensing electrode, the more likely buckling of the whole gas sensor may occur during heating and cooling periods in operation of the gas sensor. This results in stress between the different layers representing the above elements. This could cause a detachment of the different layers and a loss of electrical contact which typically may occur between the sensing electrode and the solid electrolyte.

These problems are solved by providing an adhesion promoter between the thin film and the sensing electrode, according to a preferred embodiment of the present invention. The adhesion promoter is configured to promote adhesion between the thin film electrolyte and the sensing electrode. The adhesion promoter preferably is deposited directly on the thin film ceramic or glass layer as a thin film adhesion layer or as multiple thin films, preferably by one of CVD, ALD or PVD deposition methods.

In a preferred embodiment, the adhesion promoter comprises titanium and gold, or chromium and gold. In a preferred embodiment, the adhesion promoter comprises a bilayer. Preferably, in case of the titanium and gold combination, and / or the chromium and gold combination, each combination in particular is provided in form of a bilayer. Preferably, in case of the bilayer, a diffusion barrier is comprised in between the bilayer. The diffusion barrier preferably comprises a transition metal nitride or a transition metal silicon nitride such as TiN, TaN, TaSiN layers, e.g.

Titanium preferably is deposited by means of PVD as a layer of a thickness between 5 nm and 20 nm. Gold preferably is deposited by means of PVD as a layer of a thickness between 140 nm and 160 nm, preferably 150 nm.

The adhesion promoter, and especially the materials introduced above, preferably shows one or more of a good adhesion to the thin film ceramic or glass, a good adhesion to the layer of the sensing electrode, a good permeability for the ion conducting species, such as Li⁺ or Na⁺ ions, but not limited, and optionally electrical conductivity. The permeability for the Li+ or Na+ ions can be reached by providing one or more areas between the thin film and the sensing electrode absent the adhesion promoter. These areas then represent areas permeable for the ions released from the sensing electrode. In a different embodiment, the thickness of the adhesion promoter is less than 10 nm effecting the adhesion barrier to be permeable for the ions released from the sensing electrode.

Summarizing, one of the following ways, or a combination thereof, can improve permeability for the ions released from the sensing electrode into the thin film:
- The adhesion thin film layer is structured such that one or more areas on the thin film ceramic or glass layer are not covered by it;
- The adhesion thin film layer is annealed thereby forming voids or pores such that Li+ or Na+ ions can diffuse through;
- The thickness of the adhesion thin film layer is less than 10 nm such that it will not be a barrier for Li+ or Na+ ions.

The semi-manufactured gas sensor that will be exposed to at least the annealing temperature preferably is manufactured including the following steps: The substrate is provided. The arrangement is built on the substrate by first depositing a reference electrode material on the substrate. This reference electrode material is deposited on the substrate in form of a layer or a patch building the reference electrode. In a next step, a thin film material is deposited onto the reference electrode resulting in the thin film. Preferably, the thin film material is deposited such that the reference electrode is completely encapsulated by the thin film material and the substrate.

In case of an adhesion promoter, such adhesion promoter is deposited onto the thin film in a subsequent step. The adhesion promoter may be structured including etching, for example, in order to generate one or more surface areas on the thin film not covered by the adhesion promoter. In this one or more areas, the thin film is in direct contact with the sensing electrode to be built in the subsequent step, wherein sensing electrode material is deposited onto the adhesion promoter and the one or more areas of the thin film uncovered by the adhesion promoter material if any.

In a specific embodiment, the target gas is CO2 and hence the preferred embodiment refers to a CO2 sensitive electrochemical gas sensor working in an operating temperature range between 400 and 450 °C with a Nernstian behaviour. First, a platinum reference electrode is deposited on a substrate. A platinum heater may be deposited in the same step on the substrate. The reference electrode preferably is coated with 200nm SiOx in a silane and nitrous oxide based PECVD process resulting in the non-ion-conducting thin film of SiOx. Other thicknesses of the thin film may be applied according to the above ranges, e.g. between 40 nm and 1000 nm. Then, a mixture of Li2CO3 : Au-powder in an weight ratio of 1 . 10 is deposited onto the thin film. Such electrochemical gas sensor shows a satisfying CO2 sensitivity.

According to another example that is not part of the present invention, the gas sensor is further embodied to comprise a filter. The filter preferably is selected from the group consisting of an adsorption filter, in particular an activated carbon filter, and a diffusion filter, in particular a molecular sieve, and a catalytic filter. Hence, the filter may in particular work according to one of the listed principles. In any case, the filter is permeable for the target gas, while other substance, either gaseous or solid ones present in the carrier gas, are filtered. The filtering property may be selected according to the application of the gas sensor. E.g. in case the gas sensor is used in an environment with solid particles in the ambient air, such as particulate matter, the filter property preferably is adapted to filtering out the solid particles for avoiding demining the sensing electrode or other structures of the electrochemical gas sensor. For this purpose, the filter is either arranged on top of the sensing electrode, i.e. in contact with the sensing electrode, and preferably fully covers the surface of the sensing electrode. In a different embodiment, the filter is arranged distant from the sensing electrode, i.e. a gap is provided between the filter and the sensing electrode. In such embodiment the filter and any support thereof can take the shape of a cap, for example.

According to a further example that is not part of the present invention, a method is provided for operating a gas sensor. It may be preferred that the gas sensor is to be heated to enable an electrochemical reaction in response to a target gas meeting the sensing electrode. In such a scenario, in particular the sensing electrode and the thin film, but more likely the entire arrangement plus the substrate is heated to an operating temperature. The heating then allows to measure a voltage or current between the sensing electrode and the reference electrode dependent on a concentration of the target detected by the sensing electrode. In one embodiment, the operating temperature is between 250°C and 350°C, and hence may be less than the annealing temperature applied during manufacturing. In a different embodiment, the operating temperature may be in the same range as the annealing temperature .

Other advantageous embodiments are listed in the dependent claims as well as in the description below.

### Brief Description of the Drawings

The invention will be better understood and objects other than those set forth above will become apparent from the following detailed description thereof. Such description makes reference to the annexed drawings, wherein:
Figure 1 illustrates a method for manufacturing an electrochemical gas sensor according to an embodiment of the present invention, in cut views of the electrochemical sensor;
Figure 2 illustrates an electrochemical gas sensor according to an example that is not part of the present invention, in a cut view;
Figure 3 illustrates three different top views on an adhesion promoter structure as used in an electrochemical gas sensor and a method for manufacturing an electrochemical gas sensor according to embodiments of the present invention;
Figure 4 illustrates an electrochemical gas sensor including an adhesion promoter according to Fig. 3a), and
Figure 5 shows data of an indoor field measurement of CO2 measured with a calibrated electrochemical gas sensor in accordance with an example that is not part of the present invention.

### Modes for Carrying Out the Invention

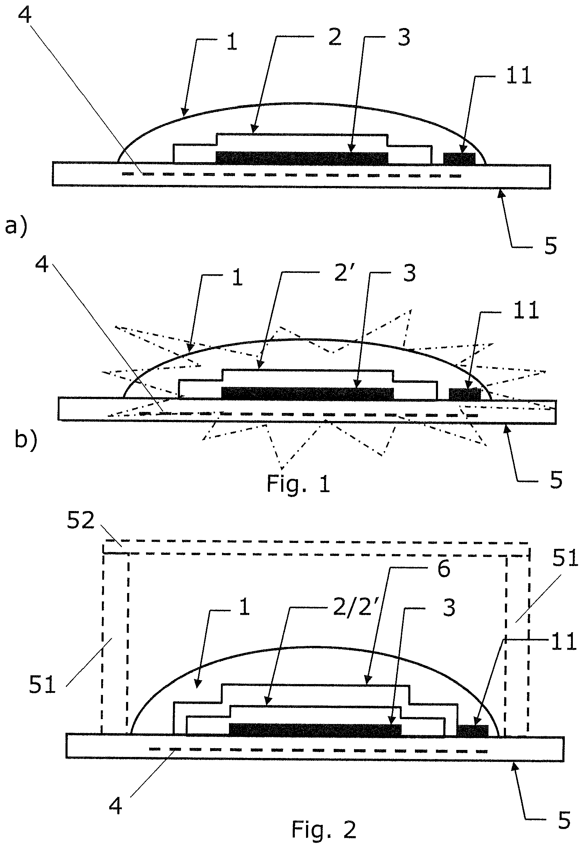

Figure 1 illustrates a method for manufacturing an electrochemical gas sensor according to an embodiment of the present invention, in two cut views a) and b) of the electrochemical sensor. In cut view 1a), a semi-manufactured electrochemical gas sensor is shown, that may be provided for final processing. The semi-manufactured electrochemical gas sensor comprises a substrate 5. The substrate 5 preferably is a semiconductor substrate. The substrate 5 supports an arrangement comprising a reference electrode 3 deposited onto the substrate 5, and hence facing the substrate 5. For this purpose, reference electrode material such as platinum is deposited on the substrate 5 and the material then is structured to form the reference electrode 3. On top of the reference electrode 3, a thin film 2 is deposited which in the present example not only fully covers the reference electrode 3 but also a small portion of the substrate 5. Hence, the reference electrode 3 is fully encapsulated by the thin film 2 and the substrate 5. This avoids the reference electrode 3 being in contact with the sensing electrode 1 which is deposited onto the thin film 2, and also parts of the substrate 5. Hence, the reference electrode 3 is out of contact with the ambient and out of contact with the sensing electrode 1, given that the substrate 5 preferably is gas tight.

In addition, a heater 4 is provided in the substrate 5. The heater 4 may be a resistive heater generating heat by applying an electric current. In case the substrate 5 is a semiconductor substrate, conductors and / or electronic circuitry may be provided in the substrate 5 for electrically connecting the heater 4, and for controlling the heater 4, including switching the heater on and off. Reference sign 11 refers to a conductor contacting the sensing electrode 1.

In the semi-manufactured state as shown in diagram 1a), the thin film 2 is electrically non-conducting, and in particular does not conduct ions that may be released from the sensing electrode material in response to an envisaged electrochemical reaction with the target gas.

In diagram 1b), the heater 4 is turned on, which is illustrated by the saw-toothed star. By doing so, the sensing electrode 1 and the thin film 2, and very likely all elements of the gas sensor, are heated to an annealing temperature T1. The thin film 2' irreversibly remains in the ion-conducting state after the heat treatment. Hence, the annealing step prepares the thin film 2 to act as ion conductor for ions released from the sensing electrode 1 to the reference electrode 3 in response of the sensing electrode 1 being exposed to the target gas. The arrangement, including the ion-conducting thin film 2' may also be heated later during operation for sensing the target gas. And it preferably is heated to an operating temperature T2 by means of the same heater 4 that is provided for the annealing temperature during manufacturing. The operating temperature T2 may be lower than the annealing temperature T1 in one embodiment.

Figure 2 illustrates an electrochemical gas sensor according to an example that is not part of the present invention, in a cut view. The illustration may either show the finally processed gas sensor, hence comprising the ion-conducting thin film 2', or may show the semi-manufactured gas sensor prior to the annealing step, and hence comprising the non-ion-conducting thin film 2.

In this example, an adhesion between the sensing electrode 1 and the thin film 2/2' is improved by a thin adhesion layer 6, also referred to as adhesion promoter 6. Such adhesion promoter 6 promotes a long term adhesion of the sensing electrode 1.

In present Figure 2, the adhesion promoter 6 is not illustrated in real scale with respect to the other elements. In this example, the adhesion promoter 6 is manufactured as a thin adhesion layer that it is permeable for the ions envisaged to be released from the sensing electrode 1. Given that there are no gaps provided in the adhesion layer 1 for allowing direct contact between the sensing electrode 1 and the thin film 2, the adhesion layer 6 itself must be permeable since on the other hand the entire surface of the thin film 2 not in touch with the substrate 5 is covered by the adhesion layer 6.

In addition, a further optional feature is illustrated by dashed lines. In case of a finally processed gas sensor, it may contain a package or encapsulation with an opening for allowing access to the sensing electrode 1. In Figure 2 such package in form of a housing is illustrated by 51. The opening in the package 51 preferably is spanned by a filter 52 which at least allows the target gas, and more preferably also gaseous medium to be investigated including the carrier gas to pass. Hence, the filter 52 may prevent particles and / or liquids to reach and impact the arrangement.

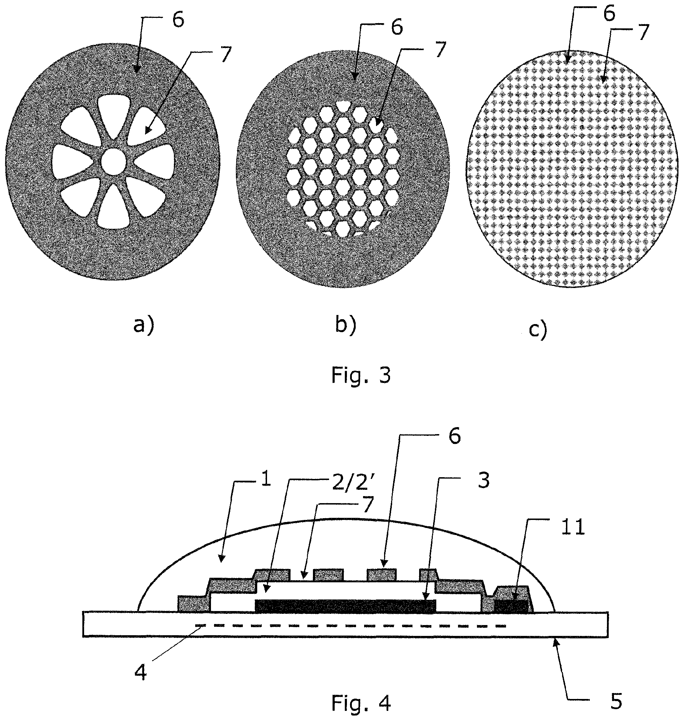

Figure 3 illustrates three different top views on an adhesion promoter 6 as used in an electrochemical gas sensor and a method for manufacturing according to embodiments of the present invention. In any of the diagrams a) to c), the area including an adhesion promoter 6 is illustrated in grey, while gaps in the adhesion promoter 6, i.e. areas where no adhesion promoter 6 is provided and hence the surface of the underlying thin film 2/2' is accessible from the top, are illustrated in white and are denoted by the reference sign 7. In those areas 7, the sensing electrode material applied on top of the adhesion promoter 6 gets into direct contact with the thin film 2 exposed in these areas 7.

The adhesion film structure shown in diagram 3c) represents a sufficient thin adhesion layer 6 as is used in the gas sensor of Figure 2. The ion permeability shall is illustrated in this diagram by multiple small holes in the adhesion layer 6, which is a simple graphical representation of the ion permeable property of the adhesion layer 6.

In diagram 3a) instead, the areas 7 uncovered by the adhesion promoter 6 are rather large and are manufactured in one or more additional steps after applying the adhesion layer 6 onto the thin film 2/2' thereby fully covering the thin film 2/2', and being of a thickness typically not permeable for the ions released from the sensing electrode 1. Such adhesion layer may, after being deposited by one of CVD, ALD or PVD, be structured by means of lithography, for example, such that the areas 7 may thereafter be released from the adhesion promoter material and constitute access areas to the underlying thin film material. As a result, in the areas 7 free from the adhesion promoter 6, the sensing electrode 1 is in direct contact with the thin film 2/2' such that ions released from the sensing electrode 1 enter the thin film 2 via the one or more areas 7.

A cut view of the gas sensor comprising the processed adhesion layer 6 as shown in diagram 3a) is illustrated in Figure 4. The three areas 7 absent of adhesion promoter material are visible in this cut view.

Diagram 3b) illustrates another structured adhesion layer 6 with processed areas 7 absent the adhesion promoter material. These areas 7 are achieved by annealing the adhesion layer 7 resulting in small areas 7 uncovered by the adhesion promoter 6.

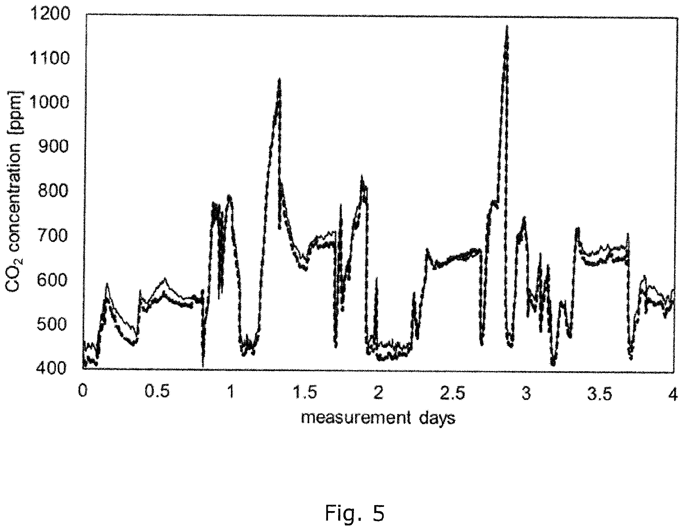

Figure 5 shows data of an indoor field measurement of CO2 measured with a calibrated electrochemical gas sensor in accordance with an example that is not part of the present invention. A calibration function (transforming a raw output voltage into a CO2 concentration) is determined beforehand in a gas mixing system. During a time period of four days the CO2 level in a meeting room was measured by said gas sensor (dashed line) and an infrared-optical (non-dispersive infrared, NDIR) reference device (thin solid line). Agreement to within 10% is found.

## Claims

1. A method for manufacturing an electrochemical gas sensor for sensing a target gas, comprising the steps of
- providing a substrate (5) supporting an arrangement comprising a thin film (2) of a thickness s ≤ 5µm arranged between a sensing electrode (1) configured to chemically interact with the target gas and a reference electrode (3) facing the substrate (5), wherein the thin film (2) is selected from the group consisting of SixOy, SixNy, SiOxNy, Al2O3, BaZrO3, and LaAlO3, or any combination thereof, wherein the sensing electrode (1) is porous at least for the target gas and comprises a compound selected from the group consisting of sodium salts, lithium salts, sodium hydroxides, and lithium hydroxides, wherein the thin film (2) is an electronically non-conducting and ionically non-conducting ceramic or glass,
- and heating the arrangement to an annealing temperature (T1) for irreversibly turning the thin film (2) into an ionic conductor by incorporating mobile ions released from the sensing electrode (1) in response to the heating.

2. The method according to claim 1, wherein an adhesion promoter (6) is provided between the thin film (2) and the sensing electrode (1),
in particular wherein the adhesion promoter (6) comprises titanium and gold, or chromium and gold, in particular in form of a bilayer,
in particular comprising a diffusion barrier in between the bilayer comprising a transition metal nitride or a transition metal silicon nitride,
in particular wherein the adhesion promoter (6) comprises one or more PVD-deposited thin films.

3. The method according to claim 2, wherein one or more areas between the thin film (2) and the sensing electrode (1) are provided absent the adhesion promoter (6) representing areas permeable for the ions released in the sensing electrode (1), and/or
wherein a thickness of the adhesion promoter (6) is less than 10 nm effecting the adhesion promoter (6) to be permeable for the mobile ions released from the sensing electrode (1).

4. The method according to any of the preceding claims, wherein the annealing temperature (T1) is greater than or equal to 300°C.

5. The method according to any of the preceding claims, wherein the thickness s of the thin film (2) is between 80nm and 500nm, in particular between 100nm and 400nm,
preferably wherein the thin film (2) is a CVD-, ALD-, or PVD-deposited thin film.

6. The method according to any of the preceding claims wherein a heater (4) is provided in or on the substrate (5), and wherein the step of heating the arrangement to the annealing temperature (T1) is effected by the heater (4).

7. The method according to any of the preceding claims, wherein the sensing electrode (1) is configured to chemically interact with the target gas by comprising a salt that is characterized as a reaction product of at least a mobile ion released form the sensing electrode (1) and the target gas,
in particular wherein the salt is selected to comprise a carbonate anion and the target gas is CO₂, and/or the salt is selected to comprise a sulfate anion and the target gas is SOx, and/or the salt is selected to comprise a nitrate/nitrite anion and the target gas is NOx.

8. The method according to any of the preceding claims 2 to 7, prior to providing the substrate (5) supporting the arrangement, building the arrangement on the substrate (5) by:
- depositing reference electrode material on the substrate (5) for building the reference electrode (3),
- depositing thin film material onto the reference electrode (3) for building the thin film (2),
- depositing an adhesion promoter (6) onto the thin film (2),
- preferably structuring the deposited adhesion promoter (6) thereby effecting one or more areas (7) on the reference electrode (3) absent adhesion promoter (6), and
depositing sensing electrode material onto the adhesion promoter (6) and areas of the reference electrode (3) free from the adhesion promoter material if any, for building the sensing electrode (1).

## Patentansprüche

1. Ein Verfahren zur Herstellung eines elektrochemischen Gassensors zum Nachweis eines Zielgases, das die folgenden Schritte umfasst
- Bereitstellen eines Substrats (5), das eine Anordnung trägt, die eine Dünnschicht (2) mit einer Dicke s ≤ 5µm umfasst, die zwischen einer Sensorelektrode (1), die so konfiguriert ist, dass sie chemisch mit dem Zielgas wechselwirkt, und einer Referenzelektrode (3), die dem Substrat (5) gegenüberliegt, angeordnet ist, wobei die Dünnschicht (2) aus der Gruppe ausgewählt ist, die aus SixOy, SixNy, Al2O3, BaZrO3 und LaAlO3 besteht, oder eine Kombination davon, wobei die Sensorelektrode (1) zumindest für das Zielgas porös ist und eine Verbindung umfasst, die aus der Gruppe ausgewählt ist, die aus Natriumsalzen, Lithiumsalzen, Natriumhydroxiden und Lithiumhydroxiden besteht, wobei die dünne Schicht (2) eine Keramik oder ein Glas ist, die bzw. das elektronisch nicht leitend und ionisch nicht leitend ist,
- und Erhitzen der Anordnung auf eine Erhitzungstemperatur (T1), um die dünne Schicht (2) irreversibel in einen Ionenleiter umzuwandeln, indem bewegliche Ionen, die als Reaktion auf das Erhitzen von der Sensorelektrode (1) freigesetzt werden, eingelagert werden.

2. Das Verfahren nach Anspruch 1, bei dem ein Haftvermittler (6) zwischen der Dünnschicht (2) und der Sensorelektrode (1) angeordnet ist,
insbesondere wobei der Haftvermittler (6) Titan und Gold oder Chrom und Gold umfasst, insbesondere in Form einer Doppelschicht,
insbesondere mit einer Diffusionsbarriere zwischen der Doppelschicht, die ein Übergangsmetallnitrid oder ein Übergangsmetall-Siliziumnitrid umfasst.
insbesondere wobei der Haftvermittler (6) eine oder mehrere durch PVD abgeschiedene Dünnschichten umfasst.

3. Das Verfahren nach Anspruch 2, wobei ein oder mehrere Bereiche zwischen der Dünnschicht (2) und der Sensorelektrode (1) frei von Haftvermittler (6) sind und Bereiche darstellen, die für in die Sensorelektrode (1) freigesetzte Ionen durchlässig sind, und/oder
wobei eine Dicke des Haftvermittler (6) weniger als 10 nm beträgt, wodurch der Haftvermittler (6) für bewegliche Ionen, die aus der Sensorelektrode (1) gebunden werden, durchlässig ist.

4. Das Verfahren nach einem der vorangehenden Ansprüche, wobei die Erhitzungstemperatur (T1) grösser oder gleich 300°C ist.

5. Das Verfahren nach einem der vorangehenden Ansprüche, wobei die Dicke s der Dünnschicht (2) zwischen 80 nm und 500 nm, insbesondere zwischen 100 nm und 400 nm, liegt,
vorzugsweise wobei die Dünnschicht (2) eine durch CVD, ALD oder PVD abgeschiedene Dünnschicht ist.

6. Das Verfahren nach einem der vorangehenden Ansprüche, wobei eine Heizvorrichtung (4) in oder auf dem Substrat (5) vorgesehen ist, und wobei der Schritt des Erwärmens der Anordnung auf die Erhitzungstemperatur (T1) mittels der Heizvorrichtung durchgeführt wird.

7. Das Verfahren nach einem der vorangehenden Ansprüche, wobei die Sensorelektrode (1) so konfiguriert ist, dass sie chemisch mit dem Zielgas wechselwirkt, indem sie ein Salz umfasst, das als Reaktionsprodukt von mindestens einem mobilen Ion, das von der Sensorelektrode (1) freigesetzt wird, und dem Zielgas gekennzeichnet ist,
insbesondere wobei das Salz so ausgewählt ist, dass es ein Carbonatanion umfasst und das Zielgas CO2 ist, und/oder das Salz so ausgewählt ist, dass es ein Sulfatanion umfasst und das Zielgas SOx ist, und/oder das Salz so ausgewählt ist, dass es ein Nitrat/Nitrit-Anion umfasst und das Zielgas NOx ist.

8. Das Verfahren nach einem der vorangehenden Ansprüche 2 bis 7,
wobei vor dem Bereitstellen des Substrats (5), das die Anordnung trägt, Aufbauen der Anordnung auf dem Substrat (5) durch:
- Aufbringen eines Referenzelektrodenmaterials auf das Substrat (5), um die Referenzelektrode aufzubauen,
- Ablagern eines Dünnschichtmaterials auf der Referenzelektrode (3), um die Dünnschicht (2) aufzubauen,
- Aufbringen eines Haftvermittlers (6) auf die Dünnschicht (2),
- vorzugsweise Strukturierung des Haftvermittlers (6), wodurch ein oder mehrere Bereiche (7) auf der Referenzelektrode (3) geschaffen werden, die frei von allfälligen Haftvermittlern sind.

## Revendications

1. Un procédé de fabrication d'un capteur de gaz électrochimique pour la détection d'un gaz cible, comprenant les étapes suivantes
- fournir un substrat (5) supportant un arrangement comprenant une couche mince (2) d'une épaisseur s ≤ 5µm disposé entre une électrode de détection (1) configurée pour interagir chimiquement avec le gaz cible et une électrode de référence (3) faisant face au substrat (5), dans lequel la couche mince (2) est sélectionné du groupe consistant en SixOy, SixNy, Al2O3, BaZrO3 et LaAlO3, ou toute combinaison de ceux-ci, dans lequel l'électrode de détection (1) est poreuse au moins pour le gaz cible et comprend un composé sélectionné du groupe consistant en sels de sodium, sels de lithium, hydroxydes de sodium et hydroxydes de lithium, dans lequel la couche mince (2) est une céramique ou un verre électroniquement non conducteur et ioniquement non conducteur,
- et chauffer l'arrangement à une température de l'échauffement (T1) pour transformer irréversiblement la couche mince (2) en un conducteur ionique en incorporant des ions mobiles libérés de l'électrode de détection (1) en réponse au chauffage.

2. Le procédé selon la revendication 1, dans lequel un promoteur d'adhésion (6) est placé entre la couche mince (2) et l'électrode de détection (1),
en particulier dans lequel le promoteur d'adhésion (6) comprend du titane et de l'or, ou du chrome et de l'or, en particulier sous la forme d'une bicouche,
en particulier comprenant une barrière de diffusion entre la bicouche comprenant un nitrure de métal de transition ou un nitrure de silicium de métal de transition.
en particulier dans lequel le promoteur d'adhésion (6) comprend une ou plusieurs couches minces déposées par PVD.

3. Le procédé selon la revendication 2, dans lequel une ou plusieurs zones entre la couche mince (2) et l'électrode de détection (1) sont dépourvues de promoteur d'adhésion (6) et représentent des zones perméables aux ions libérés dans l'électrode de détection (1), et/ou
dans lequel une épaisseur du promoteur d'adhésion (6) est inférieure à 10 nm, ce qui rend le promoteur d'adhésion (6) perméable aux ions mobiles libérés de l'électrode de détection (1).

4. Le procédé selon l'une des revendications précédentes, dans lequel la température de l'échauffement (T1) est supérieure ou égale à 300°C.

5. Le procédé selon l'une des revendications précédentes, dans lequel l'épaisseur s de la couche mince (2) est comprise entre 80 nm et 500 nm, en particulier entre 100 nm et 400 nm,
De préférence dans lequel la couche mince (2) est une couche mince déposée par CVD, ALD ou PVD.

6. Le procédé selon l'une des revendications précédentes, dans lequel un dispositif de chauffage (4) est prévu dans ou sur le substrat (5), et dans lequel l'étape de chauffage de l'arrangement à la température de l'échauffement (T1) est effectuée par le dispositif de chauffage.

7. Le procédé selon l'une des revendications précédentes, dans lequel l'électrode de détection (1) est configurée pour interagir chimiquement avec le gaz cible en comprenant un sel qui est caractérisé comme un produit de réaction d'au moins un ion mobile libéré par l'électrode de détection (1) et le gaz cible,
Particulièrement dans lequel le sel est choisi pour comprendre un anion carbonate et le gaz cible est le CO₂, et/ou le sel est choisi pour comprendre un anion sulfate et le gaz cible est le SOx, et/ou le sel est choisi pour comprendre un anion nitrate/nitrite et le gaz cible est le NOx.

8. Le procédé selon l'une des revendications précédentes 2 à 7,
avant de fournir le substrat (5) supportant l'arrangement, construire l'arrangement sur le substrat (5) en :
- déposant un matériau d'électrode de référence sur le substrat (5) pour construire l'électrode de référence,
- déposant un matériau de couche mince sur l'électrode de référence (3) pour construire la couche mince (2),
- déposant un promoteur d'adhésion (6) sur la couche mince (2),
- préférablement structurant le promoteur d'adhésion (6), par cela créant une ou plusieurs zones (7) sur l'électrode de référence (3) dépourvues de promoteur d'adhésion (6), et
déposant un matériau d'électrode de détection sur le promoteur d'adhésion (6) et sur des zones de l'électrode de référence (3) qui sont libres d'un potentiel matériau promoteur d'adhésion, afin de former l'électrode de détection (1).
